# EUROPÄISCHE PATENTSCHRIFT

(11) **EP 0 601 351 B1**
(45) Veröffentlichungstag und Bekanntmachung des Hinweises auf die Patenterteilung: **20.05.1998**
(21) Anmeldenummer: 93118286.9
(22) Anmeldetag: 11.11.1993
(51) Int. Cl.: C07D 295/088

(54) **N-(2-Sulfatoethyl)piperazin-sulfat und Verfahren zu seiner Herstellung**
N-(2-Sulphatoethyl)piperazine-sulphate and process for the production thereof
Sulfate de N-(2-sulfatoéthyl)pipérazine et son procédé de production

(30) Priorität: 21.11.1992 DE 4239182
(43) Veröffentlichungstag der Anmeldung: 15.06.1994
(73) Patentinhaber: Clariant GmbH, 65929 Frankfurt am Main (DE)
(72) Erfinder: Meier, Michael, Dr., D-60487 Frankfurt am Main (DE); Kautz, Heinz-Georg, D-63633 Birstein (DE); Schrell, Andreas, Dr., D-65929 Frankfurt am Main (DE)

(56) Entgegenhaltungen:
- EP-A- 0 546 476
- J. AMER. CHEM. SOC. Bd. 75 , 1953 Seiten 2505 - 2506 H. W. HEINE ET AL. 'The Synthesis and Alkaline Decomposition of gamma-Aminopropylsulfuric Acid'
- J. AMER. CHEM. SOC. Bd. 57 , 1935 Seite 2328 H. WENKER 'The Preparation of Ethylene Imine from Monoethanolamine'

## Beschreibung

Die Erfindung betrifft N-(2-Sulfatoethyl)piperazin-sulfat und Verfahren zu seiner Herstellung durch Umsetzung von N-(2-Hydroxyethyl)piperazin in Mischungen aus hoch oder höher konzentrierter Schwefelsäure und Oleum oder Chlorsulfonsäure, Lösen des angefallenen Reaktionsgemisches in einem niederen Alkanol und anschließende Isolation.

N-(2-Sulfatoethyl)piperazin-sulfat ist neu und kann als Mittel zur Vorbehandlung und Modifizierung von Fasermaterialien, wie synthetischen Polyamid- oder Polyurethanfasermaterialien, Wolle, Seide oder Cellulosefasermaterialien, für das anschließende Färben mit anionischen Farbstoffen eingesetzt werden [Patentanmeldung P 41 40 410.6 und P 42 24 283.5].

Gegenstand der Erfindung ist ein wirtschaftliches, technisch leicht durchführbares Verfahren zur Herstellung von N-(2-Sulfatoethyl)piperazin-sulfat in hoher Ausbeute und hoher Reinheit, indem man N-(2-Hydroxyethyl)piperazin in einer Mischung aus einer hoch- oder höher prozentigen, vorzugsweise etwa 95 bis 100 %igen, besonders bevorzugt 100 %igen Schwefelsäure, und Oleum (Lösung von Schwefeltrioxid in 100 %iger Schwefelsäure) oder Chlorsulfonsäure bei Temperaturen von etwa 80 bis etwa 250°C, vorzugsweise etwa 100° bis etwa 170°C, umsetzt, das angefallene Gemisch in einen mit Wasser mischbaren aliphatischen Alkohol, vorzugsweise Methanol, Ethanol oder Propanol, einträgt und das gebildete N-(2-Sulfatoethyl)piperazin-sulfat isoliert.

In einer zweckmäßigen Ausführungsform wird N-(2-Hydroxyethyl)piperazin in einer Mischung aus 100 %iger Schwefelsäure und Oleum, welches die stöchiometrische Menge Schwefeltrioxid enthält, oder in einer Mischung aus höherprozentiger, beispielsweise etwa 95 bis etwa 98 %iger Schwefelsäure und Chlorsulfonsäure bei Temperaturen von etwa 80 bis etwa 250°C, vorzugsweise etwa 100° bis etwa 170°C, verestert. Anschließend wird das Sulfiergemisch in einen wäßrigen, niederen Alkanol (C₁-C₃), bevorzugt Methanol oder Ethanol, eingetragen und das N-(2-Sulfatoethyl)piperazin-sulfat isoliert und getrocknet.

Beim erfindungsgemäßen Verfahren setzt man zweckmäßigerweise 100 %ige Schwefelsäure in etwa 0,8 bis etwa 3, bevorzugt etwa 1,3 bis etwa 1,7 Gewichtsteilen je Gewichtsteil N-(2-Hydroxyethyl)piperazin ein. Es können auch größere Mengen an Schwefelsäure verwendet werden. Dies ergibt jedoch keine Qualitätsvorteile und es müssen lediglich größere Mengen Schwefelsäure entsorgt werden. Anstelle 100 %iger Schwefelsäure kann auch eine Schwefelsäure mit niedrigerer Konzentration, z.B. 95 bis 99 %ige Schwefelsäure, wie 96 %ige Schwefelsäure eingesetzt werden. In diesen Fällen muß, entsprechend der zusätzlich anwesenden Wassermenge, mehr Schwefeltrioxid (im Oleum) bzw. Chlorsulfonsäure verwendet werden.

Bei der Verwendung einer Mischung aus 100 %iger Schwefelsäure und Oleum wird Schwefeltrioxid in etwa 0,3 bis etwa 0,8, bevorzugt etwa 0,4 bis etwa 0,7, besonders bevorzugt etwa 0,55 bis etwa 0,65 Gewichtsteilen je Gewichtsteil N-(2-Hydroxyethyl)piperazin eingesetzt. Bei der Verwendung einer Mischung aus höher konzentrierter Schwefelsäure und Chlorsulfonsäure wird die Chlorsulfonsäure in etwa 0,6 bis etwa 1,3, bevorzugt etwa 0,7 bis etwa 1,0, besonders bevorzugt etwa 0,80 bis etwa 0,95 Gewichtsteilen je Gewichtsteil N-(2-Hydroxyethyl)piperazin eingesetzt.

Die Menge des bevorzugt verwendeten wäßrigen Methanols bzw. Ethanols für die Fällung des N-(2-Sulfatoethyl)-piperazin-sulfats beträgt etwa 2,0 bis etwa 6,0, bevorzugt etwa 3,0 bis etwa 5,0, besonders bevorzugt etwa 3,2 bis etwa 3,8 Gewichtsteile je Gewichtsteil N-(2-Hydroxyethyl)piperazin. Der Wassergehalt des Alkohols sollte zwischen etwa 10 und etwa 30, bevorzugt zwischen etwa 15 und etwa 25 Gew.-% liegen. Die Fällung wird bei einer Temperatur von etwa 45°C begonnen und dann durch Abkühlen auf etwa 15 bis etwa 25°C zur vollständigen Kristallisation beendet. Zur Kristallisation werden etwa 0,005 bis etwa 10, bevorzugt etwa 0,01 bis etwa 5 und besonders bevorzugt etwa 0,05 bis etwa 1 Gewichtsteil N-(2-Sulfatoethyl)piperazin-sulfat je 130 Gewichtsteile N-(2-Hydroxyethyl)piperazin zum Fällungsgemisch gegeben. Es können aber auch größere Mengen Impfkristalle zugesetzt werden.

Nach der Fällung wird das N-(2-Sulfatoethyl)piperazin-sulfat isoliert und getrocknet. Es kann dann als solches verwendet werden.

Der zurückbleibende schwefelsäurehaltige, niedere aliphatische Alkohol wird zweckmäßigerweise durch Redestillation im Vakuum wiedergewonnen und in einem Folgeansatz wiederverwendet.

Die nachstehenden Beispiele dienen zur Erläuterung des erfindungsgemäßen Verfahrens, ohne es darauf zu beschränken.

### Beispiel 1

In einem 1-l-Vierhalskolben mit Rührer, Tropftrichter, Thermometer und Rückflußkühler werden 400,0 g Schwefelsäure (100 %ig) vorgelegt und 260,4 g (2,0 mol) N-(2-Hydroxyethyl)piperazin ca. 20 min.lang so zugegeben, daß die Temperatur nicht überl 150°C steigt. Anschließend dosiert man246,2 g Oleum (65 %ig) ebenfalls so zu, daß 150°C nicht überschritten werden. Das Reaktionsgemisch (906 g) wird in einen Tropftrichter überführt und dann davon ca. 1/3 in 890 g Ethanol (80 %ig) so eindosiert, daß die Temperatur 45°C nicht überschreitet. Anschließend kühlt man unter Rühren auf ca. 20°C ab, gibt gegebenenfalls 0,1 g N-(2-Sulfatoethyl)piperazin-sulfat als Impfkristalle zu und rührt heftig bis das Produkt kristallin ausfällt. Danach läßt man das restliche Reaktionsgemisch in 10 min so zulaufen, daß die Temperatur 30°C nicht übersteigt. Darauf wird unter Rühren auf 20°C abgekühlt und abgesaugt. Das Produkt wird mit 900 g Ethanol (96 %ig) nachgewaschen. Nach dem Trocknen bei 70°C und 100 mbar erhält man 608,3 g N-(2-Sulfatoethyl)piperazin-sulfat, was einer Ausbeute von 98,7 % d. Th. entspricht.
Schmp.:162°C (Phasenumwandlung?), 305°C (Zersetzung)

| | | | | |
|---|---|---|---|---|
| Berechnet für C₆H₁₆N₂O₈S₂: | C 23,2% | H 5,2% | N 9,0% | S 20,6% |
| | C 23,2% | H 5,2% | N 9,2% | S 20,2% |

¹H-NMR (D⁶-DMSO): δ = 3,34-3,56 (m; 10H), 4,06-4,12 (m; 2H), 8,6-9,2 (breit,4H)

### Beispiel 2

In einem 1-l-Vierhalskolben mit Rührer, Tropftrichter, Thermometer und Rückflußkühler werden 400,0 g Schwefelsäure (100 %ig) vorgelegt und 260,4 g (2,0 mol) N-(2-Hydroxyethyl)piperazin ca. 20 min lang so zugegeben, daß die Temperatur nicht über 150°C steigt. Anschließend dosiert man 246,2 g Oleum (65 %ig) ebenfalls so zu, daß 150°C nicht überschritten werden. Das Reaktionsgemisch (906 g) wird in einen Tropftrichter überführt und dann davon ca. 1/3 in 890 g Ethanol (80 %ig) so eindosiert, daß die Temperatur 45°C nicht überschritten wird. Anschließend kühlt man unter Rühren auf ca. 20°C ab, gibt gegebenenfalls 0,1 g N-(2-Sulfatoethyl)piperazin-sulfat als Impfkristalle zu und rührt heftig bis das Produkt kristallin ausfällt. Danach läßt man das restliche Reaktionsgemisch in 10 min so zulaufen, daß die Temperatur 30°C nicht übersteigt. Darauf wird unter Rühren auf 20°C abgekühlt und abgesaugt. Das Produkt wird mit 900 g Ethanol (96 %ig) nachgewaschen. Nach dem Trocknen bei 70°C und 100 mbar erhält man 604,9 g N-(2-Sulfatoethyl)piperazin-sulfat, was einer Ausbeute von 98,1 % d. Th. entspricht. Die spektroskopischen Daten sind mit den in Beispiel 1 angegebenen identisch.

### Beispiel 3

In einem 1-l-Vierhalskolben mit Rührer, Tropftrichter, Thermometer, Rückflußkühler und Gaseinleitung werden 206,3 g Schwefelsäure (96 %ig) vorgelegt und 130,2g (1,0 mol) N-(2-Hydroxyethyl)piperazin über ca. 20 min unter Kühlung so zugegeben, daß die Temperatur nicht über 150°C steigt. Dann werden bei 120°C unter Erwärmen 169,9 g Chlorsulfonsäure 1 h lang zudosiert. Hierbei wird ständig intensiv Stickstoff eingeleitet,weil das Reaktionsgemisch schäumt (HCl-Entwicklung). Nach beendeter Zugabe wird unter Stickstoffeinleitung bei 150°C nachgerührt bis kein Chlorwasserstoff im Abgas nachweisbar ist (ca. 1,5 h). Das Reaktionsgemisch (446,9 g) wird in einen Tropftrichter überführt und dann davon ca. 1/3 in 447 g Ethanol (80 %ig) so eindosiert, daß die Temperatur 45°C nicht überschreitet. Anschließend wird, wie in Beispiel 1 beschrieben, weiter umgesetzt. Man erhält 305,3 g N-(2-Sulfatoethyl)piperazin-sulfat, was einer Ausbeute von 99,0 % d. Th. entspricht.

Die spektroskopischen Daten und der Schmelzpunkt sind mit den in Beispiel 1 angegebenen identisch.

### Beispiel 4

In einen 1-l-Vierhalskolben mit Rührer, Tropftrichter, Thermometer und Rückflußkühler werden 200 g Schwefelsäure (100 %ig) vorgelegt und 130,2 g (1,0 mol) N-(2-Hydroxyethyl)piperazin über ca. 20 min so zugegeben, daß die Temperatur nicht über 200°C steigt. Dann werden 123,1 g Oleum (65 %ig) 1 h lang so zudosiert, daß die Temperatur 200°C nicht überschreitet. Das Reaktionsgemisch (453,2 g) wird in einen Tropftrichter überführt und dann davon ca. 1/3 in 453 g Methanol (80 %ig) so eindosiert, daß die Temperatur 45°C nicht überschreitet. Dann kühlt man unter Rühren auf ca. 25°C ab, gibt gegebenenfalls 0,1 g N-(2-Sulfatoethyl)piperazin-sulfat als Impfkristalle zu und rührt heftig bis das Produkt kristallin ausfällt. Danach läßt man das restliche Reaktionsgemisch in 10 min so zulaufen, daß die Temperatur 25°C nicht übersteigt. Anschließend wird noch 30 min bei 25°C nachgerührt und abgesaugt. Das Produkt wird mit 412 g Methanol gewaschen. Man erhält nach dem Trocknen 303,7 g N-(2-Sulfatoethyl)piperazin-sulfat, was einer Ausbeute von 98,5 % d. Th. entspricht.

Die spektroskopischen Daten sind mit den in Beispiel 1 angegebenen identisch.

## Patentansprüche

1. N-(2-Sulfatoethyl)piperazin-sulfat

2. Verfahren zur Herstellung von N-(2-Sulfatoethyl)piperazin-sulfat, dadurch gekennzeichnet, daß man N-(2-Hydroxyethyl)piperazin in einer Mischung aus hoch oder höher prozentiger Schwefelsäure und Oleum oder Chlorsulfonsäure bei Temperaturen von etwa 80°C bei etwa 250°C umsetzt, das angefallene Sulfiergemisch in einen mit Wasser mischbaren aliphatischen Alkohol einträgt und das gebildete N-(2-Sulfatoethyl)piperazin-sulfat isoliert.

3. Verfahren nach Anspruch 2, dadurch gekennzeichnet, daß man bei Temperaturen von etwa 100° bis etwa 170°C umsetzt.

4. Verfahren nach mindestens einem der Ansprüche 2 und 3, dadurch gekennzeichnet, daß die hoch oder höher konzentrierte Schwefelsäure etwa 95 bis 100 %ig ist.

5. Verfahren nach mindestens einem der Ansprüche 2 bis 4, dadurch gekennzeichnet, daß die hoch oder höher konzentrierte Schwefelsäure 100 %ig ist.

6. Verfahren nach mindestens einem der Ansprüche 2 bis 5, dadurch gekennzeichnet, daß man in etwa 0,8 bis etwa 3 Gewichtsteilen 100 %iger Schwefelsäure je Gewichtsteil N-(2-Hydroxyethyl)piperazin umsetzt.

7. Verfahren nach mindestens einem der Ansprüche 2 bis 6, dadurch gekennzeichnet, daß man in etwa 1,3 bis etwa 1,7 Gewichtsteilen 100 %iger Schwefelsäure je Gewichtsteil N-(2-Hydroxyethyl)piperazin umsetzt.

8. Verfahren nach mindestens einem der Ansprüche 2 bis 7, dadurch gekennzeichnet, daß bei der Umsetzung in einer Mischung aus hoch oder höher konzentrierter Schwefelsäure und Oleum der Gehalt des Oleums an Schwefeltrioxid etwa 0,3 bis etwa 0,8 Gewichtsteile je Gewichtsteil N-(2-Hydroxyethyl)piperazin beträgt.

9. Verfahren nach mindestens einem der Ansprüche 2 bis 8, dadurch gekennzeichnet, daß bei der Umsetzung in einer Mischung aus hoch oder höher konzentrierter Schwefelsäure und Oleum der Gehalt des Oleums an Schwefeltrioxid etwa 0,4 bis etwa 0,7 Gewichtsteile je Gewichtsteil N-(2-Hydroxyethyl)piperazin beträgt.

10. Verfahren nach mindestens einem der Ansprüche 2 bis 9, dadurch gekennzeichnet, daß bei der Umsetzung in einer Mischung aus hoch oder höher konzentrierter Schwefelsäure und Oleum der Gehalt des Oleums an Schwefeltrioxid etwa 0,55 bis etwa 0,65 Gewichtsteile je Gewichtsteil N-(2-Hydroxyethyl)piperazin beträgt.

11. Verfahren nach mindestens einem der Ansprüche 2 bis 7, dadurch gekennzeichnet, daß man bei der Umsetzung in einem Gemisch aus 100%iger Schwefelsäure und Chlorsulfonsäure etwa 0,6 bis etwa 1,3 Gewichtsteile Chlorsulfonsäure je Gewichtsteil N-(2-Hydroxyethyl)-piperazin einsetzt.

12. Verfahren nach mindestens einem der Ansprüche 2 bis 7 und 11 , dadurch gekennzeichnet, daß man bei der Umsetzung in einem Gemisch aus 100%iger Schwefelsäure und Chlorsulfonsäure etwa 0,7 bis etwa 1,0 Gewichtsteile Chlorsulfonsäure je Gewichtsteil N-(2-Hydroxyethyl)-piperazin einsetzt.

13. Verfahren nach mindestens einem der Ansprüche 2 bis 7 und 11 bis 12, dadurch gekennzeichnet, daß man bei der Umsetzung in einem Gemisch aus 100%iger Schwefelsäure und Chlorsulfonsäure etwa 0,80 bis etwa 0,95 Gewichtsteile Chlorsulfonsäure je Gewichtsteil N-(2-Hydroxyethyl)piperazin einsetzt.

14. Verfahren nach mindestens einem der Ansprüche 2 bis 13, dadurch gekennzeichnet, daß man als mit Wasser mischbarem aliphatischen Alkohol Methanol oder Ethanol verwendet.

15. Verfahren nach mindestens einem der Ansprüche 2 bis 14, dadurch gekennzeichnet, daß man das angefallene Sulfiergemisch in etwa 2,0 bis etwa 6,0 Gewichtsteile Methanol oder Ethanol je Gewichtsteil N-(2-Hydroxyethyl)piperazin einträgt.

16. Verfahren nach mindestens einem der Ansprüche 2 bis 14, dadurch gekennzeichnet, daß man das angefallene Sulfiergemisch in etwa 3,0 bis etwa 5,0 Gewichtsteile Methanol oder Ethanol je Gewichtsteil N-(2-Hydroxyethyl)piperazin einträgt.

17. Verfahren nach mindestens einem der Ansprüche 2 bis 14, dadurch gekennzeichnet, daß man das angefallene Sulfiergemisch in etwa 3,2 bis etwa 3,8 Gewichtsteile Methanol oder Ethanol je Gewichtsteil N-(2-Hydroxyethyl)piperazin einträgt.

18. Verfahren nach mindestens einem der Ansprüche 2 bis 17, dadurch gekennzeichnet, daß man das Sulfiergemisch in Methanol oder Ethanol mit einem Wassergehalt von etwa 10 bis etwa 30 Gew.-% einträgt.

19. Verfahren nach mindestens einem der Ansprüche 2 bis 18, dadurch gekennzeichnet, daß man das Sulfiergemisch in Methanol oder Ethanol mit einem Wassergehalt von etwa 15 bis etwa 25 Gew.-% einträgt.

20. Verfahren nach mindestens einem der Ansprüche 2 bis 19, dadurch gekennzeichnet, daß man dem Sulfiergemisch Impfkristalle aus N-(2-Sulfatoethyl)piperazin-sulfat zusetzt.

21. Verfahren nach mindestens einem der Ansprüche 2 bis 20, dadurch gekennzeichnet, daß man den nach der Isolation des gebildeten N-(2-Sulfatoethyl)piperazin-sulfats angefallenen schwefelsäurehaltigen Alkohol durch Destillation wiedergewinnt und in einem Folgeansatz verwendet.

## Claims

1. N-(2-Sulfatoethyl)piperazine sulfate.

2. A process for preparing N-(2-sulfatoethyl)piperazine sulfate, which comprises reacting N-(2-hydroxyethyl)piperazine in a mixture of high- or relatively high-percentage sulfuric acid and oleum or chlorosulfonic acid at temperatures of about 80°C to about 250°C, introducing the sulfation mixture formed into a water-miscible aliphatic alcohol and isolating the N-(2-sulfatoethyl)piperazine sulfate formed.

3. The process as claimed in claim 2, wherein the reaction is carried out at temperatures of about 100° to about 170°C.

4. The process as claimed in at least one of claims 2 and 3, wherein the highly or relatively highly concentrated sulfuric acid is about 95 to 100% strength sulfuric acid.

5. The process as claimed in at least one of claims 2 to 4, wherein the highly or relatively highly concentrated sulfuric acid is 100% strength sulfuric acid.

6. The process as claimed in at least one of claims 2 to 5, wherein the reaction is carried out in about 0.8 to about 3 parts by weight of 100% strength sulfuric acid per part by weight of N-(2-hydroxyethyl)piperazine.

7. The process as claimed in at least one of claims 2 to 6, wherein the reaction is carried out in about 1.3 to about 1.7 parts by weight of 100% strength sulfuric acid per part by weight of N-(2-hydroxyethyl)piperazine.

8. The process as claimed in at least one of claims 2 to 7, wherein in the reaction in a mixture of highly or relatively highly concentrated sulfuric acid and oleum the sulfur trioxide content of the oleum is about 0.3 to about 0.8 part by weight per part by weight of N-(2-hydroxyethyl)piperazine.

9. The process as claimed in at least one of claims 2 to 8, wherein in the reaction in a mixture of highly or relatively highly concentrated sulfuric acid and oleum the sulfur trioxide content of the oleum is about 0.4 to about 0.7 part by weight per part by weight of N-(2-hydroxyethyl)piperazine.

10. The process as claimed in at least one of claims 2 to 9, wherein in the reaction in a mixture of highly or relatively highly concentrated sulfuric acid and oleum the sulfur trioxide content of the oleum is about 0.55 to about 0.65 part by weight per part by weight of N-(2-hydroxyethyl)piperazine.

11. The process as claimed in at least one of claims 2 to 7, wherein in the reaction in a mixture of 100% strength sulfuric acid and chlorosulfonic acid about 0.6 to about 1.3 parts by weight of chlorosulfonic acid are used per part by weight of N-(2-hydroxyethyl)piperazine.

12. The process as claimed in at least one of claims 2 to 7 and 11, wherein in the reaction in a mixture of 100% strength sulfuric acid and chlorosulfonic acid about 0.7 to about 1.0 part by weight of chlorosulfonic acid is used per part by weight of N-(2-hydroxyethyl)piperazine.

13. The process as claimed in at least one of claims 2 to 7 and 11 to 12, wherein in the reaction in a mixture of 100% strength sulfuric acid and chlorosulfonic acid about 0.80 to about 0.95 part by weight of chlorosulfonic acid is used per part by weight of N-(2-hydroxyethyl)piperazine.

14. The process as claimed in at least one of claims 2 to 13, wherein methanol or ethanol is used as the water-miscible aliphatic alcohol.

15. The process as claimed in at least one of claims 2 to 14, wherein the sulfation mixture formed is introduced into about 2.0 to about 6.0 parts by weight of methanol or ethanol per part by weight of N-(2-hydroxyethyl)piperazine.

16. The process as claimed in at least one of claims 2 to 14, wherein the sulfation mixture formed is introduced into about 3.0 to about 5.0 parts by weight of methanol or ethanol per part by weight of N-(2-hydroxyethyl)piperazine.

17. The process as claimed in at least one of claims 2 to 14, wherein the sulfation mixture formed is introduced into about 3.2 to about 3.8 parts by weight of methanol or ethanol per part by weight of N-(2-hydroxyethyl)piperazine.

18. The process as claimed in at least one of claims 2 to 17, wherein the sulfation mixture is introduced into methanol or ethanol having a water content of about 10 to about 30% by weight.

19. The process as claimed in at least one of claims 2 to 18, wherein the sulfation mixture is introduced into methanol or ethanol having a water content of about 15 to about 25% by weight.

20. The process as claimed in at least one of claims 2 to 19, wherein N-(2-sulfatoethyl)piperazine sulfate seed crystals are added to the sulfation mixture.

21. The process as claimed in at least one of claims 2 to 20, wherein, after the N-(2-sulfatoethyl)piperazine sulfate formed has been isolated, the alcohol obtained, which contains sulfuric acid, is recovered by distillation and used in a subsequent batch.

## Revendications

1. Sulfate de N-(2-sulfatoéthyl)pipérazine.

2. Procédé de préparation du sulfate de N-(2-sulfatoéthyl)pipérazine, caractérisé en ce que l'on fait réagir la N-(2-hydroxyéthyl)pipérazine dans un mélange d'acide sulfurique à forte concentration ou à concentration plus élevée et d'oléum ou d'acide chlorosulfonique, à une température d'environ 80 °C à environ 250 °C, on introduit le mélange de sulfuration obtenu dans un alcool aliphatique miscible à l'eau et on isole le sulfate de N-(2-sulfatoéthyl)pipérazine.

3. Procédé selon la revendication 2, caractérisé en ce que l'on met en oeuvre la réaction à une température d'environ 100 °C à environ 170 °C.

4. Procédé selon au moins une des revendications 2 et 3, caractérisé en ce que l'acide sulfurique à forte concentration ou à concentration plus élevée est un acide sulfurique d'environ 95 à 100 %.

5. Procédé selon au moins une des revendications 2 à 4, caractérisé en ce que l'acide sulfurique à forte concentration ou à concentration plus élevée est un acide sulfurique à 100 %.

6. Procédé selon au moins une des revendications 2 à 5, caractérisé en ce que l'on fait réagir l'acide sulfurique à 100 % dans une quantité d'environ 0,8 à environ 3 parties en poids pour partie en poids de N-(2-hydroxyéthyl)pipérazine.

7. Procédé selon au moins une des revendications 2 à 6, caractérisé en ce que l'on fait réagir l'acide sulfurique à 100 % dans une quantité d'environ 1,3 à environ 1,7 parties en poids pour partie en poids de N-(2-hydroxyéthyl)pipérazine.

8. Procédé selon au moins une des revendications 2 à 7, caractérisé en ce que dans la réaction dans un mélange d'acide sulfurique à forte concentration ou à concentration plus élevée et d'oléum, la teneur en trioxyde de soufre dans l'oléum est d'environ 0,3 à environ 0,8 parties en poids pour partie en poids de N-(2-hydroxyéthyl)pipérazine.

9. Procédé selon au moins une des revendications 2 à 8, caractérisé en ce que dans la réaction dans un mélange d'acide sulfurique à forte concentration ou à concentration plus élevée et d'oléum, la teneur en trioxyde de soufre dans l'oléum est d'environ 0,4 à environ 0,7 parties en poids pour partie en poids de N-(2-hydroxyéthyl)pipérazine.

10. Procédé selon au moins une des revendications 2 à 9, caractérisé en ce que dans la réaction dans un mélange d'acide sulfurique à forte concentration ou à concentration plus élevée et d'oléum, la teneur en trioxyde de soufre dans l'oléum est d'environ 0,55 à environ 0,65 parties en poids pour partie en poids de N-(2-hydroxyéthyl)pipérazine.

11. Procédé selon au moins une des revendications 2 à 7, caractérisé en ce que dans la réaction dans un mélange d'acide sulfurique à 100 % et d'acide chlorosulfonique, on utilise d'environ 0,6 à environ 1,3 parties en poids d'acide chlorosulfonique pour partie en poids de N-(2-hydroxyéthyl)pipérazine.

12. Procédé selon au moins une des revendications 2 à 7 et 11, caractérisé en ce que dans la réaction dans un mélange d'acide sulfurique à 100 % et d'acide chlorosulfonique, on utilise d'environ 0,7 à environ 1,0 parties en poids d'acide chlorosulfonique pour partie en poids de N-(2-hydroxyéthyl)pipérazine.

13. Procédé selon au moins une des revendications 2 à 7 et 11 à 12, caractérisé en ce que dans la réaction dans un mélange d'acide sulfurique à 100 % et d'acide chlorosulfonique, on utilise d'environ 0,80 à environ 0,95 parties en poids d'acide chlorosulfonique pour partie en poids de N-(2-hydroxyéthyl)pipérazine.

14. Procédé selon au moins une des revendications 2 à 13, caractérisé en ce que l'on utilise du méthanol ou de l'éthanol en tant qu'alcool miscible à l'eau.

15. Procédé selon au moins une des revendications 2 à 14, caractérisé en ce que l'on introduit le mélange de sulfuration obtenu dans environ 0,2 à 6,0 parties en poids de méthanol ou d'éthanol pour partie en poids de N-(2-hydroxyéthyl)pipérazine.

16. Procédé selon au moins une des revendications 2 à 14, caractérisé en ce que l'on introduit le mélange de sulfuration obtenu dans environ 3,0 à environ 5,0 parties en poids de méthanol ou d'éthanol pour partie en poids de N-(2-hydroxyéthyl)pipérazine.

17. Procédé selon au moins une des revendications 2 à 14, caractérisé en ce que l'on introduit le mélange de sulfuration obtenu dans environ 3,2 à environ 3,8 parties en poids de méthanol ou d'éthanol pour partie en poids de N-(2-hydroxyéthyl)pipérazine.

18. Procédé selon au moins une des revendications 2 à 17, caractérisé en ce que l'on introduit le mélange de sulfuration obtenu dans du méthanol ou de l'éthanol ayant une teneur en eau d'environ 10 à environ 30 % en poids.

19. Procédé selon au moins une des revendications 2 à 18, caractérisé en ce que l'on introduit le mélange de sulfuration dans du méthanol ou de l'éthanol ayant une teneur en eau d'environ 15 à environ 25 % en poids.

20. Procédé selon au moins une des revendications 2 à 19, caractérisé en ce que l'on ajoute au mélange de sulfuration des cristaux-germes de sulfate de N-(2-sulfatoéthyl)pipérazine.

21. Procédé selon au moins une des revendications 2 à 20, caractérisé en ce que, après isolement du sulfate de N-(2-sulfatoéthyl)pipérazine formé, on récupère par distillation l'alcool contenant l'acide sulfurique et on le réutilise dans un charge ultérieure.
